# DEMANDE DE BREVET EUROPEEN

(11) **EP 2 011 474 A1**
(43) Date de publication de la demande: **07.01.2009**
(21) Numéro de dépôt: 08159102.6
(22) Date de dépôt: 26.06.2008
(51) Int. Cl.: A61K 8/22, A61K 8/25, A61K 8/81, A61Q 5/10

(54) **Composition anhydre comprenant au moins un colorant d'oxydation, au moins un complexe de peroxyde d'hydrogene et d'un polymere particulier et procédé de coloration la mettant en oeuvre**

(30) Priorité: 29.06.2007 FR 0756171; 29.06.2007 FR 0756173
(71) Demandeur: L'Oréal, 75008 Paris (FR)
(72) Inventeur: Mario, Maud, 75014, PARIS (FR); Desenne, Patricia, 92270, BOIS COLOMBES (FR); Millequant, Jean-Marie, 94100, SAINT-MAUR DES FOSSES (FR)
(74) Mandataire: Wattremez, Catherine

(57) **Abrégé**

La présente invention a pour objet une composition anhydre sous forme de pâte ou pulvérulente pour la coloration des fibres kératiniques humaines, comprenant au moins un colorant d'oxydation, au moins un complexe de peroxyde d'hydrogène et d'un polymère particulier, au moins un agent alcalin.

Elle a de même pour objet un procédé de coloration des fibres kératiniques dans lequel on met en oeuvre les étapes suivantes :
- on mélange la composition anhydre précitée, avec une composition aqueuse, avantageusement dépourvue de peroxyde d'hydrogène, et on applique la composition résultante sur les fibres kératiniques,
- on laisse pauser,
- on rince les fibres.

## Description

La présente invention a pour objet une composition anhydre pour la coloration des fibres kératiniques humaines, comprenant au moins un colorant d'oxydation, au moins un complexe de peroxyde d'hydrogène et d'un polymère particulier, au moins un agent alcalin, ainsi qu'un procédé de coloration des fibres kératiniques dans lequel on mélange la composition anhydre avec au moins de l'eau avant de l'appliquer sur lesdites fibres.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, plus particulièrement une ou plusieurs bases d'oxydation éventuellement associées à un ou plusieurs coupleurs.

Habituellement, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées, par un processus de condensation oxydative.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Le procédé de coloration employant des colorants d'oxydation est habituellement mis en oeuvre à partir de plusieurs compositions, au moins une composition tinctoriale et au moins une composition oxydante, qui sont mélangées extemporanément juste avant l'application de la composition finale sur les fibres. Il n'est en effet pas possible de stocker dans une même composition tous les ingrédients excepté l'eau, afin d'éviter la dégradation du peroxyde d'hydrogène en milieu aqueux alcalin, ce qui induit la réaction de condensation des bases d'oxydation et coupleurs.

Ainsi, selon la pratique habituelle, on stocke séparément, d'une part la ou les compositions tinctoriales, et d'autre part la composition aqueuse oxydante, qui présente un pH acide pour garantir la stabilité du peroxyde d'hydrogène, et on ne les met en contact qu'au moment de l'utilisation.

L'obligation de mettre en oeuvre plusieurs compositions entraîne tout d'abord des inconvénients inhérents au stockage de plusieurs compositions, avec une surface de stockage plus importante.

Cela entraîne par ailleurs la nécessité de doser les compositions avant de les mettre en contact. On peut proposer, pour pallier cet inconvénient, l'emploi de kits (ou dispositifs à plusieurs compartiments) mais cela complique malgré tout l'utilisation des compositions et augmente les coûts. De plus, dans certains cas, les compositions tinctoriale et oxydante, se présentent sous des formes galéniques différentes, comme par exemple des poudres, des pâtes ou des crèmes par exemple, ce qui peut compliquer le mélange et l'obtention d'une composition finale homogène.

Enfin, le fait d'utiliser des compositions oxydantes séparées nécessite de manipuler des solutions oxydantes assez concentrées.

La présente a donc pour but de proposer une composition qui comprend à la fois tous les ingrédients requis pour mettre en oeuvre un procédé de coloration d'oxydation, et qui est stable au stockage.

Cette composition permet à la fois de régler le problème du stockage en limitant le nombre de compositions à stocker, et d'en faciliter grandement la mise en oeuvre car il ne serait plus alors nécessaire de la mélanger avec une composition oxydante préalablement à son utilisation, mais simplement à de l'eau, dans les cas les plus avantageux.

Ces buts et d'autres sont atteints par la présente invention qui a donc pour objet une composition anhydre, pour la coloration des fibres kératiniques humaines, en particulier les cheveux, comprenant :
* un ou plusieurs de colorants d'oxydation ;
* un ou plusieurs complexes de peroxyde d'hydrogène et d'un polymère contenant à titre de monomère, au moins un monomère hétérocyclique vinylique ;
* un ou plusieurs agents alcalins.

Elle a de même pour objet un procédé de coloration de fibres kératiniques humaines, en particulier les cheveux, dans lequel on met en oeuvre les étapes suivantes :
- on mélange la composition selon l'invention avec une composition aqueuse et on applique la composition résultante sur les fibres kératiniques,
- on laisse pauser,
- on rince les fibres.

D'autres caractéristiques, aspects, objets et avantages de l'invention apparaîtront plus clairement à la lecture de la description qui suit.

Il est à noter que dans ce qui va suivre, et à moins d'une autre indication, les bornes d'un domaine de valeurs sont comprises dans ce domaine.

Selon l'invention, une composition est dite anhydre quand elle comprend une teneur en eau d'au plus 1 % en poids par rapport au poids de la composition. De préférence, cette teneur en eau est d'au plus 0,5 % en poids par rapport au poids de la composition.

Plus particulièrement, la teneur en eau varie de 0 à 1 % en poids et de préférence de 0 à 0,5 % en poids par rapport au poids total de la composition.

Selon un premier mode de réalisation de l'invention, la composition se présente sous forme d'une pâte.

Au sens de la présente invention, on entend par pâte anhydre, une composition anhydre présentant une viscosité supérieure à 5 poises et de préférence supérieure à 10 poises, mesurée à 25 °C et à un taux de cisaillement de 1s⁻¹ ; cette viscosité pouvant être déterminée au moyen d'un rhéomètre Coneplan.

Selon un deuxième mode de réalisation de l'invention, la composition anhydre se présente sous forme pulvérulente.

De manière avantageuse, la composition anhydre est essentiellement dépourvue de poussière (ou particules fines). Autrement dit, la distribution granulométrique des particules est telle que le taux pondéral des particules qui ont une taille inférieure ou égale à 65 micromètres (taux de fines) est avantageusement inférieur ou égal à 5%, de préférence inférieur à 2% et plus particulièrement inférieur à 1% (taille des particules évaluées au moyen d'un granulomètre RETSCH AS 200 DIGIT ; Hauteur d'oscillation : 1,25 mm / temps de tamisage : 5 minutes). De manière avantageuse, la taille des particules est comprise entre 65 µm et 2 mm.

Comme cela a été indiqué auparavant, la composition comprend un ou plusieurs colorants d'oxydation, à savoir une ou plusieurs bases d'oxydation éventuellement combinées à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

La ou les bases d'oxydation présentes dans la composition de l'invention sont en général présentes en quantité comprise allant de 0,001 à 20 % en poids environ du poids total de la composition tinctoriale, de préférence allant de 0,005 à 6 %.

Si la composition contient au moins une base d'oxydation, la composition selon l'invention contient de préférence un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthyiamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

Dans la composition de la présente invention, le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

Selon un premier mode de réalisation de l'invention, lorsque la composition se trouve sous forme de pâte, le ou les colorants d'oxydation, c'est-à-dire la ou les bases d'oxydation éventuellement combinées à un ou plusieurs coupleurs, sont choisis parmi les composés listés auparavant.

Selon un deuxième mode de réalisation de l'invention, lorsque la composition se trouve sous forme pulvérulente, le ou les colorants d'oxydation, c'est-à-dire la ou les bases d'oxydation éventuellement combinées à un ou plusieurs coupleurs, sont choisis parmi les composés benzéniques. Par composé benzénique, on entend au sens de l'invention tout composé ne comprenant dans sa structure à titre de cycle, qu'un ou plusieurs noyaux benzéniques non accolés.

Ainsi, la composition selon l'invention comprend soit une ou plusieurs bases d'oxydation benzéniques, soit un ou plusieurs coupleurs benzéniques, soit leurs combinaisons. Il est à noter qu'il n'est pas exclu que la composition comprenne en plus du ou des colorants d'oxydation benzéniques précités, une ou plusieurs bases d'oxydation non benzéniques, un ou plusieurs coupleurs non benzéniques ou leurs combinaisons.

A titre d'exemple, les bases d'oxydation benzéniques sont choisies parmi les paraphénylènediamines, les bis-phénylalkylènediamines, les para-aminophénols, les ortho-aminophénols et leurs sels d'addition.

Parmi les coupleurs benzéniques, on peut notamment citer les méta-phénylènediamines, les méta-aminophénols, les méta-diphénols, ainsi que leurs sels d'addition.

Selon une variante de ce mode de réalisation, la composition peut comprendre également une ou plusieurs bases d'oxydation additionnelles non benzéniques, et de façon avantageuse, choisies parmi les bases hétérocycliques. Parmi les bases hétérocycliques convenables, on peut citer à titre d'exemple, les dérivés pyridiniques, les dérivés pyrimidiniques et les dérivés pyrazoliques.

Selon une autre variante de ce mode de réalisation, la composition peut comprendre également un ou plusieurs coupleurs additionnels non benzéniques. Plus particulièrement, ces derniers sont choisis parmi les coupleurs naphtaléniques ou hétérocycliques.

Dans tous ces cas, on pourra se reporter aux listes précédemment données concernant ces composés.

Conformément à un mode de réalisation avantageux de l'invention, la composition est dépourvue de colorant direct.

La composition comprend en outre un ou plusieurs complexes de peroxyde d'hydrogène et d'un polymère contenant à titre de monomère au moins un monomère hétérocyclique vinylique.

Plus particulièrement, le monomère hétérocyclique vinylique est choisi parmi les monomères comprenant un hétérocycle ayant 4 à 6 chaînons, éventuellement condensé à un cycle benzénique et comporte de 1 à 4 hétéroatomes intracycliques identiques ou non ; le nombre d'hétéroatomes intracycliques étant inférieur à celui des chaînons de l'hétérocycle. De préférence, le nombre d'hétéroatomes intracycliques est de 1 ou 2.

Plus particulièrement, le ou les hétéroatomes sont choisis parmi le soufre, l'oxygène, l'azote, de préférence parmi l'azote et l'oxygène. Conformément à un mode de réalisation encore plus avantageux de l'invention, le monomère comprend au moins un atome d'azote intracyclique.

L'hétérocycle vinylique peut éventuellement être substitué par un ou plusieurs groupements alkyle en C₁-C₄, de préférence en C₁-C₂.

De préférence, le monomère hétérocyclique est choisi parmi les monomères N-vinyliques.

Parmi les monomères envisageables, on peut citer les monomères suivants, éventuellement substitués : la N-vinylpyrrolidone, le vinylcaprolactame, la N-vinylpipéridone, la N vinyl 3-morpholine, la N-vinyl-4-oxazolinone, la 2-vinylpyridine, la 4-vinylpyridine, la 2-vinylquinoline, le 1-vinylimidazole, le 1-vinylcarbazole. De préférence, le monomère est la N-vinylpyrrolidine éventuellement substituée.

Conformément à un mode de réalisation particulièrement avantageux de l'invention, le polymère est un homopolymère.

Il n'est cependant pas exclu de mettre en oeuvre un copolymère. Dans un tel cas, le ou les comonomères sont choisis parmi l'acétate de vinyle, les acides (méth)acryliques, les amides (meth)acryliques, les esters d'alkyle en C₁-C₄ d'acide (méth)acrylique substitués ou non.

Le polymère intervenant dans ce complexe peut de plus être soluble ou insoluble dans l'eau. De préférence, il est soluble dans l'eau. Il peut présenter des poids moléculaires moyens variables, de préférence compris entre 10³ et 3.10⁶ g/mol, de préférence entre 10³ et 2.10⁶ g/mol. Il est également possible d'employer des mélanges de tels polymères.

De manière avantageuse, ledit complexe comprend de 10 à 30% en poids de peroxyde d'hydrogène, plus particulièrement de 13 à 25 % en poids et de préférence de 18 à 22% en poids, par rapport au poids total du complexe.

Selon une variante encore plus avantageuse de l'invention, dans ce complexe, le rapport molaire entre le ou les monomères hétérocycliques vinyliques et le peroxyde d'hydrogène va de 0,5 à 2, de préférence de 0,5 à 1.

Ce complexe se présente avantageusement sous forme d'une poudre substantiellement anhydre, c'est-à-dire contenant moins de 5% en poids d'eau.

Des complexes de ce type sont décrits notamment dans US 5008106, US 5077047, EP 832846, EP 714919, DE 4344131, DE 19545380.

A titre d'exemples de complexes, on peut citer par exemple les produits du type Peroxydone K-30, Peroxydone K-90, Peroxydone XL-10 ainsi que les complexes formés avec le peroxyde d'hydrogène et l'un des polymères suivants de type Plasdone K-17, Plasdone K-25, Plasdone K-29/32, Plasdone K-90, Polyplasdone INF-10, Polyplasdone XL-10, Polyplasdone XL, Plasdone S-630, Styleze 2000 Terpolymer, série des Ganex copolymers, commercialisés par la société ISP.

La composition anhydre selon l'invention contient avantageusement de 0,1 à 50 % en poids de complexe de polymère contenant à titre de monomère au moins un monomère hétérocyclique vinylique et de peroxyde d'hydrogène, de préférence de 1 à 30 % en poids par rapport au poids total de la composition.

La composition anhydre comprend en outre un ou plusieurs agents alcalins.

Le ou les agents alcalins sont plus particulièrement choisis parmi l'ammoniac, les silicates, comme les silicates et métasilicates, les phosphates, hydrogénophosphates, carbonates ou hydrogénocarbonates de métaux alcalins ou alcalino-terreux, tels que le lithium, le sodium, le potassium, le magnésium, le calcium, le baryum, et leurs mélanges.

De préférence, le ou les agents alcalins sont choisis parmi les silicates, les carbonates de métaux alcalins, et leurs mélanges.

La concentration en agents alcalins représente avantageusement de 0,01 à 40 % en poids, et de préférence de 0,1 à 30 % en poids du poids total de la composition.

De préférence, la composition selon l'invention comprend également un ou plusieurs sels d'ammonium comme le chlorure d'ammonium, le sulfate d'ammonium, le phosphate d'ammonium ou le nitrate d'ammonium.

Conformément à un mode de réalisation encore plus avantageux de l'invention, le sel d'ammonium est le chlorure d'ammonium.

La concentration en sel(s) d'ammonium, s'ils sont présents, est avantageusement comprise entre 0,01 et 40 % en poids par rapport au poids total de la composition, de préférence de 0,1 à 30 % en poids par rapport au poids total de la composition.

Dans le cas où la composition se présente sous forme de pâte, la composition selon l'invention comprend en outre un ou plusieurs liquides inertes organiques.

Par liquide inerte, on entend au sens de la présente invention tout composé capable d'écoulement à température ambiante, généralement entre 15 °C et 40 °C, et à pression atmosphérique, sous l'action de son propre poids.

Inerte signifie que le liquide ne réagit pas au moins dans les conditions de stockage, avec les ingrédients de la composition.

A titre d'exemples de liquide inerte, on peut citer les polydécènes de formule C₁₀ₙH_{[(20n)+2]} dans laquelle n varie de 3 à 9 et de préférence de 3 à 7, les esters et en particulier les esters d'alcools gras ou d'acides gras, les esters ou di-esters de sucres d'acides gras en C₁₂-C₂₄, ou les esters cycliques, les éthers cycliques, les huiles de silicone, les huiles minérales, les huiles végétales ou les huiles animales, ou leurs mélanges.

Les composés de formule C₁₀ₙH_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo^{®} 366 NF Polydecene par la société Amoco Chemical, ceux vendus sous la dénomination Nexbase® 2002 FG, 2004 FG, 2006 FG et 2008 FG par la société Fortum.

En ce qui concerne les esters on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates. Parmi ces esters, on préfère plus particulièrement utiliser le palmitate d'isopropyle, le myristate d'isopropyle, le stéarate d'octyl dodécyle et l'isononaoate d'isononyle.
- les esters de monoalcools linéaires ou ramifiés en C₃-C₈, avec des acides gras bifonctionnels en C₈-C₂₄, ces derniers pouvant être linéaires ou ramifiés, saturés ou insaturés, comme par exemple le di-ester isopropylique de l'acide sébacique, appelé aussi sébaçate de di-isopropyle,
- les esters de monoalcools linéaires ou ramifiés en C₃-C₈, avec des acides bifonctionnels en C₂-C₈, ces derniers pouvant être linéaires ou ramifiés, saturés ou insaturés, comme par exemple l'adipate de di-octyle et le maléate de di-caprylyle,
- l'ester d'un acide trifonctionnnel comme le citrate de tri-éthyle.

En ce qui concerne les esters et di-esters de sucres d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

En ce qui concerne les ethers cycliques et esters cycliques, conviennent notamment la γ-butyrolactone, le diméthyl isosorbide, ou le diisopropyl isosorbide.

Les huiles de silicone peuvent aussi être employées comme liquide organique inerte.

Plus particulièrement, les huiles de silicone convenables sont des fluides de silicones liquides de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s, DC-8566 amino fluid, DC 245 fluid, par la société Dow Corning.

Les huiles minérales peuvent aussi être utilisées en tant que liquide inerte organique, comme par exemple l'huile de paraffine, la vaseline.

Les huiles végétales peuvent aussi convenir, et notamment l'huile d'avocat, l'huile d'olive ou la cire liquide de jojoba, l'huile de camellia, de même que les huiles animales telles que la lanoline.

On peut encore utiliser des solvants apolaires comme notamment les dérivés dicapryliques apolaires, et plus particulièrement le dicapryl carbonate, le dicaprylether.

De préférence, le liquide inerte organique est choisi parmi les polydécènes de formule C₁₀ₙH_{[(20n)+2]} dans laquelle n varie de 3 à 9 et de préférence de 3 à 7, les esters d'alcools gras ou d'acides gras, l'huile de vaseline, l'huile de paraffine, et leurs mélanges.

La teneur en liquide(s) inerte(s) organique(s) varie, dans la composition, avantageusement de 10 à 50 % en poids par rapport au poids de la composition, et de préférence de 20 à 50 % en poids par rapport au poids de la composition.

La composition peut éventuellement comprendre au moins un sel peroxygéné.

Les sels peroxygénés sont plus particulièrement choisis parmi les persulfates, les perborates et les percarbonates de métaux alcalins ou alcalino-terreux, comme le sodium, le potassium, le magnésium.

Selon une variante préférée de l'invention, la composition comprend, à titre de sels peroxygénés, des persulfates et parmi ceux-ci principalement les persulfates de sodium et de potassium.

Habituellement, la teneur en sel(s) peroxygéné(s) s'il est présent, représente de 1 à 70 % en poids, plus particulièrement de 10 à 70 % en poids et de préférence de 20 à 60 % en poids par rapport au poids total de ladite composition.

La composition selon l'invention peut de plus comprendre un ou plusieurs polymères épaississant.

Avantageusement les polymères épaississants sont choisis parmi les polymères suivants :
(i) les polymères amphiphiles non ioniques comportant au moins une chaîne grasse et au moins un motif hydrophile ;
(ii) les polymères amphiphiles anioniques comportant au moins un motif hydrophile, et au moins un motif à chaîne grasse ;
(iii) les homopolymères d'acide acrylique réticulés;
(iv) les homopolymères réticulés d'acide 2-acrylamido-2-méthyl-propane sulfonique et leurs copolymères réticulés d'acrylamide partiellement ou totalement neutralisés ;
(v) les homopolymères d'acrylate d'ammonium ou les copolymères d'acrylate d'ammonium et d'acrylamide ;
(vi) les homopolymères de diméthylaminoéthyl-méthacrylate quaternisé par le chlorure de méthyle ou les copolymères de diméthylamino-éthylméthacrylate quaternisé par le chlorure de méthyle et d'acrylamide ;
(vii) les polysaccharides ;
(viii) la gommes de scléroglucane (biopolysaccharide d'origine microbienne),
(ix) les gommes issues d'exudats végétaux telles que les gommes arabique, Ghatti, Karaya et Tragacanthe;
(x) les celluloses et dérivés.

Il est à noter que dans le cas de la présente invention, les polymères épaississants ont un rôle sur la viscosité de la composition prête à l'emploi, c'est-à-dire de la composition résultant du mélange de la composition anhydre selon l'invention avec au moins de l'eau.

Selon l'invention, les polymères amphiphiles sont plus particulièrement des polymères hydrophiles capables, dans le milieu de la composition, et plus particulièrement un milieu aqueux, de s'associer réversiblement entre eux ou avec d'autres molécules.

Leur structure chimique comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 8, de préférence au moins 10 atomes de carbone, de préférence de 10 à 30 atomes de carbone, en particulier de 12 à 30 atomes de carbone et plus préférentiellement de 18 à 30 atomes de carbone. Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères épaississants amphiphiles non ioniques comportant au moins une chaîne grasse et au moins un motif hydrophile sont choisis de préférence parmi :
(1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
(2) les hydroxypropylguars modifiés par des groupements comportant au moins une chaîne grasse tel que le produit ESAFLOR HM 22 (chaîne alkyle en C₂₂) vendu par la société LAMBERTI, les produits MIRACARE XC95-3 (chaîne alkyle en C₁₄) et RE205-1 (chaîne alkyle en C₂₀) vendus par la société RHONE POULENC.
(3) les uréthanes polyéthers comportant au moins une chaîne grasse telle que des groupes alkyle ou alcényle en C₈-C₃₀, comme les produits DAPRAL T 210 et DAPRAL T 212 vendus par la société AKZO.
(4) les copolymères de vinyl pyrrolidone et de monomères hydrophobes à chaîne grasse ; on peut citer à titre d'exemple :
   - les produits ANTARON V216 ou GANEX V216 (copolymère vinylpyrrolidone /hexadécène) vendu par la société I.S.P.
   - les produits ANTARON V220 ou GANEX V220 (copolymère vinylpyrrolidone /eicosène) vendu par la société I.S.P.
(5) les copolymères de méthacrylates ou d'acrylates d'alkyles en C₁-C₆ et de monomères amphiphiles comportant au moins une chaîne grasse tels que par exemple le copolymère méthacrylate de méthyle/acrylate de stéaryle oxyéthyléné vendu par la société GOLDSCHMIDT sous la dénomination ANTIL 208.
(6) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

Parmi les polymères amphiphiles anioniques comportant au moins un motif hydrophile, et au moins un motif à chaîne grasse, on peut citer ceux comportant au moins un motif éther d'allyl à chaîne grasse et au moins un motif hydrophile constitué par un monomère anionique insaturé éthylénique, plus particulièrement par un acide carboxylique vinylique et tout particulièrement par un acide acrylique, un acide méthacrylique ou leurs mélanges, le motif éther d'allyl à chaîne grasse correspondant au monomère de formule (1) suivante :

CH₂ = CR'-CH₂-O-Bₙ-R (1)

dans laquelle R' désigne H ou CH₃, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant 8 à 30 atomes de carbone, de préférence 10 à 24, et plus particulièrement encore de 12 à 18 atomes de carbone.
Un motif de formule (I) plus particulièrement préféré selon la présente invention est un motif dans lequel R' désigne H, n est égal à 10, et R désigne un radical stéaryle (C₁₈).

Des polymères amphiphiles anioniques de ce type sont décrits et préparés, selon un procédé de polymérisation en émulsion, dans le brevet EP-0216479 B2.

Parmi ces polymères amphiphiles anioniques, on préfère particulièrement selon l'invention, les polymères formés à partir de 20 à 60% en poids d'acide acrylique et/ou d'acide méthacrylique, de 5 à 60% en poids de (méth)acrylates d'alkyls inférieurs, de 2 à 50% en poids d'éther d'allyle à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyle, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

Les polymères amphiphiles anioniques peuvent être également choisis parmi ceux comportant au moins un motif hydrophile de type acide carboxylique insaturé oléfinique, et au moins un motif hydrophobe exclusivement de type ester d'alkyl (C₁₀-C₃₀) d'acide carboxylique insaturé, utilisés selon l'invention, sont choisis de préférence parmi ceux dont le motif hydrophile de type acide carboxylique insaturé oléfinique correspond au monomère de formule (2) suivante : 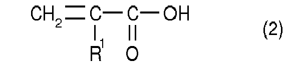 formule dans laquelle, R¹ désigne H ou CH₃ ou C₂H₅, c'est-à-dire des motifs acide acrylique, acide méthacrylique ou acide éthacrylique et dont le motif hydrophobe de type ester d'alkyl (C₁₀-C₃₀) d'acide carboxylique insaturé correspond au monomère de formule (3) suivante : 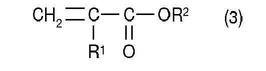 formule dans laquelle, R¹ désigne H ou CH₃ ou C₂H₅ (c'est-à-dire des motifs acrylates, méthacrylates ou éthacrylates) et de préférence H (motifs acrylates) ou CH₃ (motifs méthacrylates), R² désignant un radical alkyle en C₁₀-C₃₀, et de préférence en C₁₂-C₂₂.

Des esters d'alkyle (C₁₀-C₃₀) d'acides carboxyliques insaturés comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères amphiphiles anioniques de ce type sont par exemple décrits et préparés, selon les brevets US-3 915 921 et 4 509 949.

Les polymères amphiphiles anioniques utilisables dans le cadre de la présente invention peuvent désigner plus particulièrement des polymères formés à partir d'un mélange de monomères comprenant :
(i) essentiellement de l'acide acrylique, un ester de formule (3) suivante : 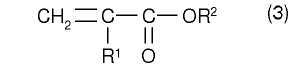 dans laquelle R¹ désigne H ou CH₃, R² désignant un radical alkyle ayant de 12 à 22 atomes de carbone, et un agent réticulant, tels que par exemple ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant,
(ii) essentiellement de l'acide acrylique et du méthacrylate de lauryle tel que celui formé à partir de 66% en poids d'acide acrylique et 34% en poids de méthacrylate de lauryle.

Ledit réticulant est un monomère contenant un groupe CH₂=CH< avec au moins un autre groupement polymérisable dont les liaisons insaturées sont non conjuguées l'une par rapport à l'autre. On peut notamment citer les polyallyléthers tels que notamment le polyallylsucrose et le polyallylpentaérythritol.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.C. sous la dénomination COATEX SX.

Parmi les homopolymères d'acide acrylique réticulés utilisables dans le cadre de la présente invention, on peut citer ceux réticulés par un éther allylique d'alcool de la série du sucre, comme par exemple les produits vendus sous les noms CARBOPOLS 980, 981, 954, 2984 et 5984 par la société GOODRICH ou les produits vendus sous les noms SYNTHALEN M et SYNTHALEN K par la société 3 VSA ;

Parmi les homopolymères réticulés d'acide 2-acrylamido-2-méthyl-propane sulfonique, on peut citer ceux décrits dans la demande EP-A-0815828 (faisant partie intégrante du contenu de la description). Parmi les copolymères réticulés d'acide 2-acrylamido-2-méthyl-propane sulfonique et d'acrylamide partiellement ou totalement neutralisés (par une base telle que la soude, de la potasse ou une amine) on peut citer en particulier le produit décrit dans l'exemple 1 du document EP-A-503 853 (faisant partie intégrante du contenu de la description).

Parmi les homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST (ils sont décrits et préparés dans les documents FR 2 416 723, USP2798053 et USP 2 923 692) ;

Parmi les homopolymères de diméthylaminoéthyl-méthacrylate quaternisé par le chlorure de méthyle, on peut citer les produits vendus sous les noms SALCARE 95 et SALCARE 96 par la société ALLIED COLLOIDS. Parmi les copolymères de diméthylamino-éthylméthacrylate quaternisé par le chlorure de méthyle et d'acrylamide, on peut citer le produit SALCARE SC92 vendu par ALLIED COLLOIDS ou le produit PAS 5194 vendu par HOECHST (ils sont décrits et préparés dans le document EP-A-395.282).

Parmi les polysaccharides, on peut citer les polysaccharides anioniques, tels que les pectines, les carrageenanes ; les polysaccharides cationiques comme le chitosan ; les polysaccharides non ioniques comme les gommes de guar non modifiées.

Plus particulièrement, les gommes de guar non ioniques non modifiées sont par exemple les produits vendus sous la dénomination VIDOGUM GH 175 par la société UNIPECTINE et sous la dénomination JAGUAR C par la société MEYHALL.

Les gommes de guar non ioniques utilisables selon l'invention sont de préférence modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2 et.

De telles gommes de guar non ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

La gomme de scléroglucane (biopolysaccharide d'origine microbienne), les gommes issues d'exsudats végétaux telles que les gommes arabique, Ghatti, Karaya et Tragacanthe, sont bien connues de l'homme de l'art et décrites notamment dans l'ouvrage de Robert L. DAVIDSON intitulé "Handbook of Water soluble gums and resins" édité chez Mc Graw Hill Book Company (1980).

En ce qui concerne les celluloses et dérivés, on peut citer notamment les celluloses ethers non ioniques, comme les hydroxyalkylcelluloses (alkyle en C₂-C₃), avec par exemple le Natrosol 250HHR ; les celluloses cationiques telles que le Polyquaternium-10 (dénomination CTFA) avec par exemple le produit Ucare Polymer FR400, le polyquaternium-4 (dénomination CTFA) ; les celluloses anioniques avec les carboxyalkylcellulose (alkyle en C₁-C₂).

Les polymères épaississants sont utilisés de préférence en une quantité pouvant varier de 0,01 à 15% en poids par rapport au poids de la composition, et de préférence de 0,1 à 10% en poids par rapport au poids de la composition.

La composition selon l'invention peut comprendre un ou plusieurs agents tensioactifs.

Par agent tensioactif, on entend au sens de la présente invention, un agent comportant au moins un groupe hydrophile et au moins un groupe lipophile dans sa structure, et qui est de préférence apte à diminuer la tension superficielle de l'eau, et ne comportant dans sa structure, comme motifs répétitifs éventuels, que des motifs oxyde d'alkylène et/ou des motifs sucres et/ou des motifs siloxane. De préférence, le groupement lipophile est une chaîne grasse comprenant de 8 à 30 atomes de carbone.

Cet agent tensioactif peut être choisi parmi les agents tensioactifs anioniques, amphotères, non ioniques, cationiques ou leurs mélanges.

Les tensioactifs convenant à la mise en oeuvre de la présente invention sont notamment les suivants :

### (i) Tensioactif(s) anionique(s) :

A titre d'exemple de tensioactifs anioniques utilisables, seuls ou mélanges, dans le cadre de la présente invention, on peut citer notamment les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

### (ii) Tensioactif(s) non ionique(s) :

Les agents tensioactifs non ioniques sont, eux aussi, des composés bien connus en soi (voir notamment à cet égard "Handbook of Surfactants" par M. R. PORTER, éditions Blackie & Son (Glasgow and London), 1991, pp 116-178).

Ils peuvent être notamment choisis parmi :
- les alcools gras polyéthoxylés, polypropoxylés ou polyglycérolés, les alpha-diols polyéthoxylés, polypropoxylés ou polyglycérolés, les alkylphénols polyéthoxylés, polypropoxylés ou polyglycérolés, le nombre de groupements oxyde d'éthylène ou oxyde de propylène pouvant aller notamment de 2 à 50 et le nombre de groupements glycérol pouvant aller notamment de 2 à 30 ;
- les copolymères d'oxyde d'éthylène et de propylène, les condensats d'oxyde d'éthylène et de propylène sur des alcools gras ;
- les amides gras polyéthoxylés ayant de préférence de 2 à 30 moles d'oxyde d'éthylène, les amides gras polyglycérolés comportant plus particulièrement en moyenne 1 à 5 groupements glycérol et en particulier 1,5 à 4 ;
- les esters d'acides gras du sorbitan oxyéthylénés ayant plus particulièrement de 2 à 30 moles d'oxyde d'éthylène ; les esters d'acides gras du sucrose, les esters d'acides gras du polyéthylèneglycol,
- les alkylpolyglycosides,
- les dérivés de N-alkyl glucamine, les oxydes d'amines tels que les oxydes d'alkyl amines ou les oxydes de N-acylaminopropylmorpholine ;
- et leurs mélanges

Plus particulièrement, dans les composés ci-dessus, la chaîne grasse ou la chaîne alkyle ou le groupement acyle comprend par exemple 8 à 30 atomes de carbone.

On notera que les alkylpolyglycosides constituent des tensioactifs non ioniques rentrant particulièrement bien dans le cadre de la présente invention.

### (iii) Tensioactif(s) amphotère(s) :

Les agents tensioactifs amphotères peuvent être notamment :
- des dérivés d'amines secondaires ou tertiaires aliphatiques, dans lesquels le radical aliphatique est une chaîne linéaire ou ramifiée comportant 8 à 24 atomes de carbone et contenant au moins un groupe anionique hydrosolubilisant (par exemple carboxylate, sulfonate, sulfate, phosphate ou phosphonate) ;
- des alkylbétaïnes, les alkylsulfobétaïnes, les alkylamidoalkyl (C₁-C₆) bétaïnes ou les alkylamidoalkyl (C₁-C₆) sulfobétaïnes, avec une chaîne alkyle comprenant de 8 à 24 atomes de carbone.

Parmi les dérivés d'amines, on peut citer les produits commercialisés sous les dénominations MIRANOL, tels que décrits dans les brevets US-2 528 378 et US-2 781 354 et de structures :

R₂ -CONHCH₂CH₂ -N(R₃)(R₄)(CH₂COO⁻) (III)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (IV)

dans laquelle :
- B représente -CH₂CH₂OX', C représente -(CH₂)_{z} -Y', avec z = 1 ou 2,
- X' désigne le groupement -CH₂CH₂-COOH ou un atome d'hydrogène
- Y' désigne -COOH ou le radical -CH₂-CHOH-SO₃H
- R₅ désigne un radical alkyle d'un acide R₉-COOH présent dans l'huile de coprah ou dans l'huile de lin hydrolysée, un radical alkyle, linéaire ou ramifié, notamment en C₇-C₁₇, comprenant éventuellement au moins un insaturation éthylénique.

Ces composés sont classés dans le dictionnaire CTFA, 5ème édition, 1993, sous les dénominations Disodium Coco-ampho-diacetate, Disodium Lauro-ampho-diacetate, Disodium Capryl-ampho-diacetate, Disodium Caprylo-ampho-diacetate, Disodium Coco-ampho-dipropionate, Disodium Lauro-ampho-dipropionate, Disodium Capryl-ampho-dipropionate, Disodium Caprylo-ampho-dipropionate, Lauro-ampho-dipropionic acid, Coco-ampho-dipropionic acid.

A titre d'exemple on peut citer le cocoamphodiacetate commercialisé sous la dénomination commerciale MIRANOL C2M concentré par la société RHODIA CHIMIE.

### (iv) Tensioactif(s) cationique(s) :

Les tensioactifs cationiques peuvent être choisis parmi :
(A) les sels d'ammonium quaternaires de la formule générale (V) suivante : 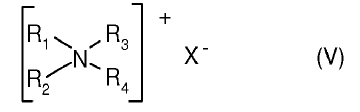 dans laquelle X- est un anion choisi par exemple dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate, et
   (1) les radicaux R₁ à R₃, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R₄ désigne un radical alkyle, linéaire ou ramifié, comportant de 16 à 30 atomes de carbone.
      De préférence le tensioactif cationique est un sel (par exemple chlorure) de béhényl triméthyl ammonium, et
   (2) les radicaux R₁ et R₂, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone ;
      R₃ et R₄, identiques ou différents, désignent un radical alkyle, linéaire ou ramifié, comportant de 12 à 30 atomes de carbone, ledit radical comprenant au moins une fonction ester ou amide.
      R₃ et R₄ sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
(B) les sels d'ammonium quaternaire de l'imidazolinium, comme par exemple celui de formule (VI) suivante : 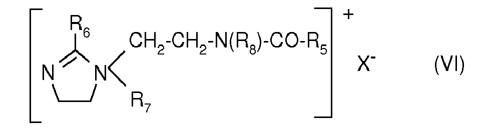 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion notamment choisi dans le groupe des halogénures, phosphates, acétates, lactates, alkylsulfates, alkyl-ou-alkylarylsulfonates.
   De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
(C) les sels de diammonium quaternaire de formule (VII) : 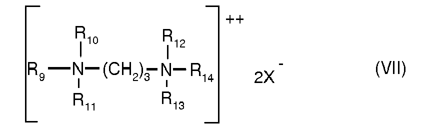 dans laquelle R₉ désigne un radical aliphatique comportant de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion notamment choisi dans le groupe des halogénures, acétates, phosphates, nitrates et méthylsulfates.
   De tels sels de diammonium quaternaire comprennent notamment le dichlorure de propanesuif diammonium.
(D) les sels d'ammonium quaternaire contenant au moins une fonction ester de formule (VIII) suivante : 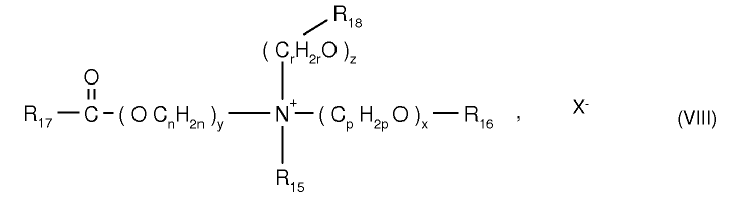 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical R₁₉-CO-- les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical R₂₁-CO-- les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X- est un anion simple ou complexe, organique ou inorganique ;
   sous réserve que la somme x + y + z vaut de 1 à 15, que lorsque x vaut 0 alors R₁₆ désigne R₂₀ et que lorsque z vaut 0 alors R₁₈ désigne R₂₂ ;
   ou leurs mélanges.

On utilise plus particulièrement les sels d'ammonium de formule (VIII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical R₁₉-CO-;
   - les radicaux méthyle, éthyle ou hydrocarbonés en C₁₄C₂₂;
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical R₂₁-CO-;
   - l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Le tensioactif lorsqu'il est présent, représente plus particulièrement de 0,01 % et 60 % en poids par rapport au poids total de la composition, de préférence entre 0,5 % et 30 % en poids et encore plus préférentiellement entre 1 % et 20 % en poids.

La composition anhydre selon l'invention peut en outre comprendre un ou plusieurs polymères substantifs, cationiques ou amphotères.

Le caractère substantif (c'est à dire l'aptitude au dépôt sur les cheveux) des polymères est classiquement déterminé au moyen du test décrit par Richard J. Crawford, Journal of the Society of Cosmetic Chemists, 1980, 31 - (5) - pages 273 à 278 (révélation par colorant acide Red 80).

Ces polymères substantifs sont notamment décrits dans la littérature dans la demande de brevet EP-A-0557203.

Parmi les polymères substantifs du type homopolymère ou copolymère d'halogénure de diméthyldiallylammonium utilisables selon l'invention, on peut citer en particulier :
- les polymères de chlorure de diallyldiméthylammonium comme les polyquaternium-6 (Merquat 100 de la société Calgon)
- les copolymères de chlorure de diallyldiméthylammonium et d'acide acrylique comme celui de proportions (80/20 en poids) vendu sous la dénomination Merquat 280 par la société Calgon;
- les copolymères du chlorure de diméthyldiallylammonium et de l'acrylamide vendus sous les dénominations Merquat 550 et Merquat S par la société Merck.

Parmi les polymères substantifs du type polymère d'halogénure de méthacryloyloxyéthyltriméthylammonium utilisables selon l'invention, on peut citer en particulier les produits qui sont dénommés dans le dictionnaire CTFA (5ème édition, 1993) "Polyquaternium 37" , "Polyquaternium 32" et "Polyquaternium 35" , qui correspondent respectivement, en ce qui concerne le "Polyquaternium 37", au poly(chlorure de méthacryloyloxyéthyltriméthyl-ammonium) réticulé, en dispersion à 50% dans de l'huile minérale, et vendu sous la dénomination Salcare SC95 par la société Allied Colloids, en ce qui concerne le "Polyquaternium 32", au copolymère réticulé de l'acrylamide et du chlorure de méthacryloyloxyéthyltriméthylammonium (20/80 en poids), en dispersion à 50% dans de l'huile minérale, et vendu sous la dénomination Salcare SC92 par la société Allied Colloids, et en ce qui concerne le "Polyquaternium 35", au méthosulfate du copolymère de méthacryloyloxyéthyltriméthylammonium et de méthacryloyloxyéthyldiméthylacétylammonium, vendu sous la dénomination Plex 7525L par la société Rohm GmbH.

Les polymères substantifs du type polyammonium quaternaire utilisables selon l'invention sont les suivants :
- les polymères préparés et décrits dans le brevet français 2 270 846, constitués de motifs récurrents répondant à la formule (I) suivante : 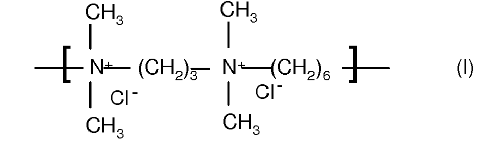 notamment ceux dont le poids moléculaire, déterminé par chromatographie par perméation de gel, est compris entre 9500 et 9900 ;
- les polymères préparés et décrits dans le brevet français 2 270 846, constitués de motifs récurrents répondant à la formule (II) suivante : 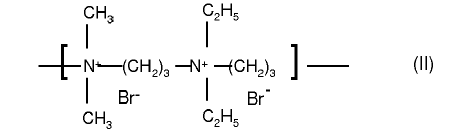 notamment ceux dont le poids moléculaire, déterminé par chromatographie par perméation de gel, est d'environ 1200 ;
- les polymères décrits et préparés dans les brevets US 4 157 388, 4 390 689, 4 702 906, 4 719 282, et constitués de motifs récurrents répondant à la formule (III) suivante : 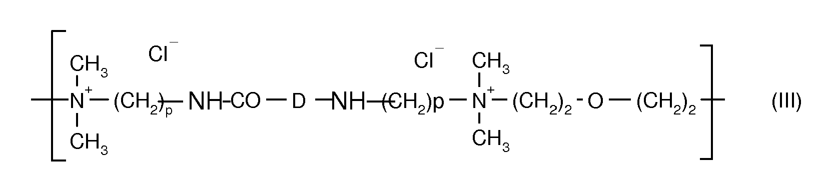 dans laquelle p désigne un nombre entier variant de 1 à 6 environ, D peut être nul ou peut représenter un groupement -(CH₂)ᵣ-CO- dans lequel r désigne un nombre égal à 4 ou à 7, et notamment ceux dont la masse moléculaire est inférieure à 100 000, de préférence inférieure ou égale à 50 000 ; de tels polymères sont notamment vendus par la société Miranol sous les dénominations "Mirapol A15", "Mirapol AD1", "Mirapol AZ1" et "Mirapol 175" ;

Parmi les polymères de Vinylpyrrolidone (PVP) à motifs cationiques utilisables conformément à l'invention, on peut citer en particulier :
a) les polymères de Vinylpyrrolidone comportant des motifs Méthacrylate de diméthylaminoéthyle ; on peut citer parmi ceux-ci :
   - le copolymère Vinylpyrrolidone / Méthacrylate de diméthylaminoéthyle (20/80 en poids) vendu sous la dénomination commerciale COPOLYMER 845 par la société I.S.P.
   - les copolymères Vinylpyrrolidone / Méthacrylate de diméthylaminoéthyle quaternisés par du sulfate de diéthyle, vendus sous les dénominations GAFQUAT 734, 755, 755 S et 755 L par la société I.S.P.
   - les PVP / Méthacrylate de diméthylaminoéthyle / Polyuréthane hydrophile, vendus sous la dénomination commerciale PECOGEL GC-310 par la société U.C.I.B. ou encore sous les dénominations AQUAMERE C 1031 et C 1511 par la société BLAGDEN CHEMICALS,
   - les PVP / Méthacrylate de diméthylaminoéthyle / Oléfine en C8 à C16, quaternisés ou non quarternisés, vendus sous les dénominations GANEX ACP 1050 à 1057, 1062 à1069, 1079 à 1086, par la société I.S.P.
   - le PVP / Méthacrylate de diméthylaminoéthyle / Vinylcaprolactame, vendu sous la dénomination GAFFIX VC 713 par la société I.S.P.
b) les polymères de Vinylpyrrolidone comportant des motifs Méthacrylamidopropyltriméthylammonium (M.A.P.T.A.C.), parmi lesquels on peut citer notamment :
   - les copolymères Vinylpyrrolidone / M.A.P.T.A.C., vendus sous les dénominations commerciales GAFQUAT ACP 1011 et GAFQUAT HS 100 par la société I.S.P.
c) les polymères de Vinylpyrrolidone comportant des motifs Méthylvinylimidazolium, et parmi lesquels on peut citer plus particulièrement :
   - les PVP / Chlorure de méthylvinylimidazolium, vendus sous les dénominations LUVIQUAT FC 370, FC 550, FC 905, HM 552 par la société B.A.S.F.
   - le PVP / Chlorure de méthylvinylimidazolium / Vinylimidazole, vendu sous la dénomination LUVIQUAT 8155 par la société B.A.S.F.
   - le PVP / Méthosulfate de méthylvinylimidazolium, vendu sous la dénomination LUVIQUAT MS 370 par la société B.A.S.F.

Parmi les polysiloxanes cationiques on peut notamment citer ceux décrits dans la demande de brevet EP-A-0557203, de la page 8 ligne 48 à la page 11 ligne 9, et plus particulièrement encore les produits comprenant l'Amodiméthicone" (dénomination C.T.F.A.) de formule (IV) suivante :

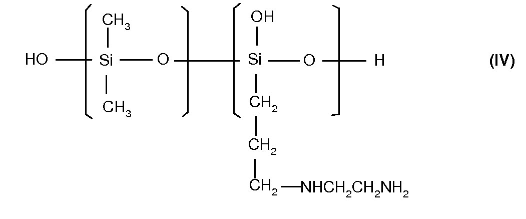

La concentration en polymère substantif cationique ou amphotère peut varier entre 0,01 et 10 % environ par rapport au poids de la composition, et de préférence entre 0,1 et 5 % en poids par rapport au poids de la composition.

La composition selon l'invention peut également comprendre divers additifs classiquement utilisés en cosmétique.

La composition conforme à la présente invention peut ainsi comprendre des agents épaississants minéraux ou organiques, et en particulier des charges telles que des argiles ; des liants tels que la vinylpyrrolidone ; des lubrifiants comme les stéarates de polyol ou les stéarates de métaux alcalins ou alcalino-terreux ; des silices hydrophiles ou hydrophobes ; des pigments ; des agents matifiants ou opacifiants comme les oxydes de titane ; des agents antioxydants comme l'acide érythorbique ; des agents réducteurs comme le métabisulfite de sodium ; des agents de pénétration, des agents séquestrants, comme l'éthylènediamine tétraacétique ou ses sels ; des agents absorbeurs d'humidité comme les silices amorphes, certains polyacrylates réticulés ou modifiés par des groupements hydrophobes comme par exemple les produits Luquasorb 1010 de BASF, Polytrap 6603 Adsorber de AMCOL; des tampons ; des agents dispersants ; des agents filmogènes ; des agents conservateurs ; des vitamines ; des parfums ; des céramides, des agents de conditionnement autres que les polymères substantifs mentionnés ci-dessus.

Il est à noter que la composition peut également comprendre une ou plusieurs substances colorées dont la nature et/ou la concentration ne permettent pas de colorer les fibres kératiniques traitées.

La composition conforme à l'invention peut également comprendre des agents de contrôle du dégagement d'oxygène tels que le carbonate ou l'oxyde de magnésium.

Les additifs et les agents de contrôle du dégagement d'oxygène tels que définis précédemment peuvent être présents en quantité comprise pour chacun d'eux entre 0,01 et 40 % en poids, de préférence entre 0,1 et 30 % en poids par rapport au poids total de la composition.

De préférence, la composition anhydre sous forme de pâte est constituée par un mélange d'au moins une poudre et d'au moins un liquide inerte organique.

Dans le cas où la composition se trouve sous forme pulvérulente, elle peut aussi comprendre des composés permettant une agglomération des particules de poudre, hydrosolubles ou non, polymériques ou non, comme par exemple l'alcool polyvinylique, les copolymères polyoxyde d'éthylène / polyoxyde de propylène (Pluronic®, Symperonic®).

A titre purement illustratif, leur teneur ne dépasse en général pas 5 % en poids par rapport au poids de la composition.

La composition anhydre selon l'invention sous forme de pâte peut être avantageusement préparée en dispersant, sous action mécanique, l'ensemble des composés pulvérulents dans le liquide inerte organique, dans lequel on a préalablement dispersé ou mélangé les autres composés liquides de la composition.

On peut également préparer la pâte par extrusion, en introduisant les phases liquides et solides de la composition dans un extrudeur, puis en les mélangeant à une température inférieure à 25°C à l'aide d'un système bi-vis co-rotatives composé d'éléments de transport et de malaxage.

Le procédé de coloration selon l'invention consiste mélanger la composition anhydre qui vient d'être décrite avec une composition aqueuse et à appliquer la composition résultante sur les fibres kératiniques,
- on laisse pauser,
- on rince les fibres.

La composition aqueuse peut être simplement de l'eau.

La composition aqueuse peut éventuellement comprendre au moins un solvant polaire. Parmi les solvants polaires utilisables dans cette composition, on peut citer des composés organiques liquides à température ambiante (25°C) et au moins partiellement miscibles à l'eau.

A titre d'exemple, on peut citer plus particulièrement les alcanols tels que l'alcool éthylique, l'alcool isopropylique, les alcools aromatiques comme l'alcool benzylique, et l'alcool phényléthylique, ou encore des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol.

Plus particulièrement, si un ou plusieurs solvants sont présents, leur teneur respective dans la composition aqueuse varie de 0,5 à 20% et de préférence, de 2 à 10% en poids par rapport au poids de ladite composition aqueuse.

De préférence, la composition aqueuse ne comprend pas de peroxyde d'hydrogène.

Si toutefois elle en comprend, la concentration pondérale en peroxyde d'hydrogène est de 2 à 12% en peroxyde d'hydrogène, de préférence de 2 à 6 %.

Elle peut contenir en outre des agents stabilisants du peroxyde d'hydrogène tels que notamment le pyrophosphate de sodium, le stannate de sodium et le salicylate de sodium.

Lorsque ladite composition aqueuse comprend du peroxyde d'hydrogène, elle présente de préférence un pH inférieur à 7. Le pH acide garantit la stabilité du peroxyde d'hydrogène dans la composition. Il peut être obtenu à l'aide d'agents acidifiants tels que par exemple l'acide chlorhydrique, l'acide acétique, l'acide éthydronique, l'acide phosphorique, l'acide lactique ou l'acide borique, et il peut être ajusté classiquement par ajout soit d'agents alcalinisants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

Quelle contienne ou non du peroxyde d'hydrogène, la composition aqueuse peut contenir en outre des agents conservateurs, des colorants, des parfums, des agents antimousse, ainsi que des agents séquestrants tels que par exemple l'acide éthylènediamine tétraacétique (EDTA) ou le Pentasodium Pentetate (dénomination CTFA).

Elle peut d'ailleurs se présenter sous la forme d'une solution, d'une émulsion ou un gel.

Généralement, le taux de dilution de la composition selon l'invention est tel que la composition résultante puisse être facilement appliquée sur les fibres kératiniques à colorer, tout en restant localisée à l'endroit où elle a été appliquée, afin d'éviter les problèmes causés par les coulures de composition hors de la zone à traiter.

Plus particulièrement, le taux de dilution (composition anhydre / composition aqueuse ; rapport exprimé en poids) est compris entre 2/1 et 1/10.

Le pH du mélange résultant est habituellement compris entre 7 et 12. De préférence, le pH dudit est habituellement compris entre 7,5 et 11.

Une fois le mélange réalisé pour obtenir la composition prête à l'emploi, celle-ci est appliquée sur les fibres kératiniques humaines, sèches ou humides.

Le temps de pause est en général de l'ordre de 1 minute à 1 heure, de préférence de 10 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, lavées au shampooing, rincées à nouveau à l'eau puis séchées ou laissées à sécher.

Les exemples qui suivent servent à illustrer l'invention sans toutefois en limiter la portée.

### EXEMPLE 1

On prépare la composition suivante :

| **Ingrédients** | **Quantités (%g)** |
|---|---|
| Métasilicate de sodium | 10 |
| Chlorure d'ammonium | 3 |
| EDTA | 1 |
| Carboxymethyl cellulose de sodium | 5 |
| Diéthylhexyl sulfosuccinate de sodium et benzoate de sodium | 3 |
| lauryl sulfate de sodium | 8 |
| Acide ascorbique | 0.2 |
| Paraphénylène diamine | 0.73 |
| Méta amino phénol | 0.11 |
| 2-méthyl-5-hydroxyéthylamoniphénol | 1.1 |
| Distéarate de glycol | 12.86 |
| PVP/H₂O₂ (Peroxydone K30) | 15 |
| Paraffine | 15 |
| Lanoline | 25 |

Cette pâte mélangée à température ambiante à de l'eau en dilution 1 poids de pâte pour 2 poids d'eau.

La composition résultante est appliquée sur une mèche de cheveux naturels à 90 % de blanc, à température ambiante pendant une durée de 30 minutes.

A l'issue du temps de pause, la mèche est rincée à l'eau puis lavée avec un shampooing standard et rincée de nouveau puis séchée.

On obtient une mèche de couleur violacée.

### EXEMPLE 2

| **Ingrédients** | **Quantités (%g)** |
|---|---|
| Silicate de sodium | 15 |
| Chlorure d'ammonium | 3 |
| EDTA | 1 |
| Amidon carboxyméthylé sous forme de sel de sodium (Primogel) | 1 |
| Gomme de xanthane | 3 |
| Steareth-100/PEG-136/HDI Copolymer | 3 |
| Lauryl sulfate de sodium | 5 |
| Stéarate de magnésium | 4 |
| Acide ascorbique | 0.2 |
| Paraphénylène diamine | 0.73 |
| Méta amino phénol | 0.11 |
| 2-méthyl-5-hydroxyéthylamoniphénol | 1.1 |
| Distéarate de glycol | 7.86 |
| PVP/H₂O₂ (Peroxydone K30) | 15 |
| Myristate d'isopropyle | 20 |
| Lanoline | 20 |

Cette pâte mélangée à température ambiante à de l'eau en dilution 1 poids de pâte pour 2 poids d'eau.

La composition résultante est appliquée sur une mèche de cheveux naturels à 90 % de blanc, à température ambiante pendant une durée de 30 minutes.

A l'issue du temps de pause, la mèche est rincée à l'eau puis lavée avec un shampooing standard et rincée de nouveau puis séchée.

On obtient une mèche de couleur violacée.

### EXEMPLE 3

| **Ingrédients** | **Quantités (%g)** |
|---|---|
| Disilicate de sodium | 33,12 |
| Métasilicate de sodium | 1,8 |
| Chlorure d'ammonium | 3,5 |
| Carbonate de sodium | 5,7 |
| EDTA | 0,7 |
| Argile | 5,5 |
| Carbopol ET2020 (commercialisé par la société Noveon) | 6,9 |
| Amidon carboxyméthylé sous forme de sel de sodium (Primogel commercialisé par la société AVEBE) | 4,1 |
| Cétéaryl sulfate de sodium | 2,7 |
| Stéarate de magnésium | 4,1 |
| Polyvinylpyrrolidone | 4,1 |
| Oxyde de titane | 4,1 |
| Polyquaternium-22 | 0,3 |
| PVP/H₂O₂ (Peroxydone K-30 commercialisé par la société ISP) | 20,7 |
| Paraphénylène diamine | 1 |
| Méta-aminophénol | 0,15 |
| 2-méthyl 5-β-hydroxyéthyl aminophénol | 1,5 |
| Lanoline | 25 |

Cette composition sous forme de poudre est mélangée à de l'eau avec un rapport poids composition / poids eau de 1/2.

Elle est ensuite appliquée sur des mèches de cheveux à 90% de blancs ou châtain clair pendant 30 minutes à température ambiante.

A l'issue de ce traitement, les mèches sont rincées à l'eau, puis séchées.

On obtient des mèches de cheveux teints avec un reflet de couleur violacée.

## Revendications

1. Composition anhydre pour la coloration des fibres kératiniques humaines, comprenant,
* un ou plusieurs colorants d'oxydation ;
* un ou plusieurs complexes de peroxyde d'hydrogène et d'un polymère contenant à titre de monomère, au moins un monomère hétérocyclique vinylique ;
* un ou plusieurs agents alcalins.

2. Composition anhydre selon la revendication 1, **caractérisée en ce que** la composition se trouve sous forme de pâte.

3. Composition selon la revendication 1, **caractérisée en ce que** le ou les précurseurs de colorants d'oxydation sont choisis parmi les composés benzéniques.

4. Composition selon l'une des revendications 1 ou 3, **caractérisée en ce que** la composition se trouve sous forme pulvérulente.

5. Composition selon l'une quelconque des revendications précédentes, **caractérisée en ce que** le monomère hétérocyclique vinylique est choisi parmi les monomères N-vinyliques.

6. Composition l'une quelconque des revendications précédentes, **caractérisée en ce que** le monomère hétérocyclique vinylique est la vinylpyrrolidone.

7. Composition selon l'une quelconque des revendications précédentes, **caractérisée en ce qu'**elle contient de 0,1 à 50 % en poids de complexe de peroxyde d'hydrogène et de polymère, par rapport au poids total de la composition.

8. Procédé de coloration de fibres kératiniques humaines, **caractérisé en ce que** l'on met en oeuvre les étapes suivantes :
- on mélange la composition selon l'une quelconque des revendications précédentes, avec une composition aqueuse et on applique la composition résultante sur les fibres kératiniques,
- on laisse pauser,
- on rince les fibres.

9. Procédé selon la revendication précédente, **caractérisé en ce que** la composition aqueuse ne comprend pas de peroxyde d'hydrogène.

10. Procédé selon l'une des revendications 8 ou 9, **caractérisé en ce que** la composition aqueuse est de l'eau.

11. Procédé selon la revendication 8, **caractérisé en ce que** la composition aqueuse comprend du peroxyde d'hydrogène à une concentration pondérale allant de 2 à 12 %.
